# EUROPÄISCHE PATENTANMELDUNG

(11) **EP 3 124 959 A1**
(43) Veröffentlichungstag der Anmeldung: **01.02.2017**
(21) Anmeldenummer: 16181127.8
(22) Anmeldetag: 26.07.2016
(51) Int. Cl.: G01N 21/88

(54) **PROBABILISTISCHE FEHLERERKENNUNG IN VERLEGTEN FASERBÄNDCHEN**

(30) Priorität: 31.07.2015 DE 102015009728
(71) Anmelder: Airbus Defence and Space GmbH, 82024 Taufkirchen (DE)
(72) Erfinder: Engel, Franz, 81539 München (DE)
(74) Vertreter: Isarpatent

(57) **Zusammenfassung**

Ein erstes erfindungsgemäßes Verfahren dient einem probabilistischen Erkennen von Fehlern bei auf einem Faserverbundwerkstück verlegten Materialbändchen. Es umfasst ein Erfassen mindestens eines Oberflächenprofils (100a, 100b, 100c, 100d) eines Werkstücks mit verlegten Materialbändchen und ein Auffinden eines Oberflächenprofileintrags (201, 201 a, 201 c', 201 c", 201 c'") in einer Datenbank (200). Der Oberflächenprofileintrag liefert dabei eine Zuordnung des Oberflächenprofils (100a, 100c) zu einem Fehlertyp und einer Trefferwahrscheinlichkeit dafür, dass dem erfassten Oberflächenprofil ein Fehler des Fehlertyps zugrunde liegt. Ein zweites erfindungsgemäßes Verfahren dient einem Anlegen einer entsprechend geeigneten Datenbank (200).

Eine erfindungsgemäße Prüfeinrichtung dient einer Kontrolle von Faserverbundwerkstücken, und eine erfindungsgemäße Anlage dient einer automatisierten Herstellung von Faserverbundbauteilen.

## Beschreibung

Die vorliegende Erfindung betrifft ein Verfahren zum probabilistischen Erkennen von Fehlern bei auf einem Faserverbundwerkstück verlegten Materialbändchen mittels einer Datenbank. Die Erfindung betrifft weiterhin ein Verfahren zum Anlegen einer entsprechend geeigneten Datenbank. Schließlich betrifft die Erfindung ein computerlesbares Speichermedium mit Instruktionen zum Ausführen eines Verfahrens zum probabilistischen Erkennen von Fehlern, eine Prüfeinrichtung zur Kontrolle eines Faserverbundwerkstücks und eine Anlage zur automatisierten Herstellung von Faserverbundbauteilen.

Faserverbundkunststoffe werden in vielen Bereichen eingesetzt. Insbesondere finden kohlenstofffaserverstärkte Kunststoffe beispielsweise Verwendung in industriellen Bauteilen, in der Luft- und Raumtechnik und in Sportgeräten. Die Herstellung derartiger Faserverbundkunststoffe kann insbesondere automatische Schichtlegeprozesse umfassen. Mit Hilfe eines Legekopfes werden dabei trockene oder vorimprägnierte Fasern (beispielsweise Kohlenstofffasern) in schmalen Bändchen nebeneinander sowie in mehreren Schichten übereinander in einem formgebenden Werkzeug verlegt.

Dabei kann es zu verschiedenen Mängeln kommen. Beispielsweise können bei einem fehlerhaften Legen einer Schicht unerwünschte Verdrehungen, Überlappungen, Spalte oder Materialfalten auftreten. Daneben kann Fremdmaterial in oder auf die Schichten gelangen; derartiges unerwünschtes Material kann aus der Anlage selbst herrühren (beispielsweise Materialabrieb im Legekopf) oder bei einem offenen Werkzeug als Verschmutzung aus der Umgebung eindringen.

Zur Vermeidung nachteiliger Konsequenzen werden gemäß dem Stand der Technik gelegte Schichten nach jeder abgeschlossenen Lage von Mitarbeitern optisch auf derartige Mängel hin untersucht. Dazu kann das formgebende Werkzeug von den entsprechenden Kontrolleuren betreten werden, zum Teil gibt es auch Hebesysteme, mit denen die Arbeiter über die Form schweben können, um das gelegte Material zu besichtigen.

Für eine Entscheidung darüber, wie bei einem festgestellten Fehler vorgegangen werden sollte, ist ferner eine Typisierung des Mangels vorteilhaft, mit der also eine Art des Fehlers bestimmt, also beispielsweise festgestellt wird, dass eine Verdrehung, ein Materialspalt oder Ähnliches vorliegt. Auch diese Typisierung erfolgt nach dem Stand der Technik durch optische Kontrolle durch Mitarbeiter, die eine entsprechende Zuordnung eines erkannten Fehlers vornehmen.

Die genannten Kontroll- und Typisierungsmechanismen sind aufwendig, beanspruchen viel Zeit und sind oft ungenau. Zudem erfordern insbesondere große Kontrollflächen oft eine systematische Blickführung, die oft schwer einzuhalten ist, so dass Mängel leicht übersehen werden können. Schließlich können das Werkzeug betretende oder über dem Material geführte Personen oder auch die entsprechenden Hebesysteme die verlegten Schichten selbst beschädigen oder verschmutzen.

Die vorliegende Erfindung hat daher die Aufgabe, eine Technik bereitzustellen, mittels der Fehler bei auf einem Faserverbundwerkstück verlegten Materialbändchen unter Vermeidung der vorgenannten Nachteile erkannt werden können.

Die Aufgabe wird gelöst durch Verfahren gemäß den Ansprüchen 1 und 12, durch mindestens ein computerlesbares Medium gemäß Anspruch 10, durch eine Prüfeinrichtung gemäß Anspruch 11 und eine Anlage automatisierten Herstellung von Faserverbundbauteilen gemäß Anspruch 14. Bevorzugte Ausführungsformen sind in den Unteransprüchen offenbart.

Ein erstes erfindungsgemäßes Verfahren dient einem probabilistischen Erkennen von Fehlern bei auf einem Faserverbundwerkstück verlegten Materialbändchen; das Material der Materialbändchen kann dabei insbesondere nicht ausgehärteten kohlenstofffaserverstärkten Kunststoff umfassen. Ein derartiges Verfahren umfasst ein Erfassen mindestens eines Oberflächenprofils eines Werkstücks mit verlegten Materialbändchen. Das Oberflächenprofil kann dabei lediglich einen lokal eingegrenzten Bereich der Oberfläche des Werkstücks betreffen, insbesondere einen Abschnitt einer Linie; besonders bevorzugt sind Ausführungsformen, bei denen das Oberflächenprofil einen Linienabschnitt betrifft, der mindestens ein verlegtes Materialbändchen kreuzt. Das genannte erste erfindungsgemäße Verfahren umfasst weiterhin ein Auffinden eines Oberflächenprofileintrags in einer Datenbank; der Oberflächenprofileintrag liefert (d.h. ermöglicht, z.B. umfasst) dabei eine Zuordnung des erfassten Oberflächenprofils zu einem Fehlertyp und zu (einem Wert) einer Wahrscheinlichkeit dafür, dass dem erfassten Oberflächenprofil ein Fehler des (so zugeordneten) Fehlertyps tatsächlich zugrunde liegt; diese Wahrscheinlichkeit (bzw. dieser Wahrscheinlichkeitswert) wird zur Unterscheidbarkeit von anderen hier betrachteten Wahrscheinlichkeiten in dieser Schrift als "Trefferwahrscheinlichkeit" bezeichnet (weil sie eine Wahrscheinlichkeit dafür angibt, dass die Zuordnung des Oberflächenprofils zu dem Fehlertyp auf die Realität zutrifft).

Der Begriff "Wahrscheinlichkeit" wird in dieser Schrift dem allgemeinen Sprachgebrauch entsprechend gelegentlich mit einem jeweils ihr zugewiesenen Wert identifiziert. Die zugrundeliegende Zuweisung kann beispielsweise auf Grundlage von bekannten Daten (z.B. Erfahrungswerten), mindestens einem statistischen Verfahren und/oder maschinellem Lernen erfolgt sein.

Die Trefferwahrscheinlichkeit ist vorzugsweise positiv (d.h. größer als 0); insbesondere umfasst die Datenbank vorzugsweise keine Zuordnungen mit probabilistischer Unmöglichkeit, so dass die Größe der Datenbank relativ klein gehalten werden kann und ein aufgefundener Eintrag besonders nützlich ist.

Ein erfindungsgemäßes Verfahren ermöglicht somit ein automatisiertes Erkennen von Fehlern, welche die verlegten Materialbändchen aufweisen können; das Erkennen umfasst dabei ein probabilistisches Typisieren eines jeweiligen Fehlers. So können aufwendige und fehleranfällige Inspektionen eines Werkstücks durch Mitarbeiter zumindest reduziert und bei einem entsprechenden Fehler gleichwohl für den jeweiligen Fehlertyp geeignete Maßnahmen getroffen werden, die ggf. spezifisch auf den Fehlertyp abgestimmt sein können.

Gemäß einer bevorzugten Ausführungsform umfasst das Erfassen des Oberflächenprofils bei einem derartigen ersten erfindungsgemäßen Verfahren ein Abtasten mindestens eines Oberflächenabschnitts des Werkstücks mit mindestens einer Sensoreinrichtung. Die Sensoreinrichtung kann beispielsweise einen Laser-Lichtschnitt-Sensor (bzw. Triangulationssensor) umfassen. Das Abtasten kann, muss aber nicht flächig erfolgen. Insbesondere kann das Abtasten entlang einer Linie oder Kurve erfolgen, die vorzugsweise mindestens ein Materialbändchen, bevorzugter mehrere Materialbändchen kreuzt. Auf diese Weise können insbesondere Fehler an den Übergängen der Materialbändchen, beispielsweise Spalte oder Überlappungen erfasst werden.

Gemäß einer vorteilhaften Variante umfasst ein erstes erfindungsgemäßes Verfahren ein Charakterisieren des mindestens einen (bzw. jedes) erfassten Oberflächenprofils durch eine (jeweilige) Abfolge, die Angaben über bei dem Oberflächenprofil auftretende Stufenrichtungen, Stufenbreiten und/oder Stufenhöhen umfasst (und die vorzugsweise Rückschlüsse auf eine zugehörige Stufenanordnung (z.B. Reihenfolge) ermöglicht). Vorzugsweise ist damit die Abfolge eine digitale Repräsentation des realen Oberflächenprofils. Die Stufenrichtungen können dabei z.B. binär als "steigend" bzw. "fallend" (in Bezug auf den jeweiligen Untergrund) charakterisiert werden, oder es können Winkelbereiche angegeben sein, in die ein Abschnitt des Oberflächenprofils fällt. Vorteilhafterweise erfolgt das Charakterisieren automatisch, insbesondere kann es ein automatisches Generieren der Abfolge umfassen.

Mit der Abfolge kann für das Oberflächenprofil ein vom Datenumfang her kleinerer Repräsentant mit vorgegebener Struktur in dem weiteren Ablauf des Verfahrens verwendet werden. Insbesondere kann das Auffinden des Oberflächenprofileintrags vereinfacht und beschleunigt werden, indem es auf Grundlage der Abfolge erfolgt.

Gemäß einer bevorzugten Ausführungsform umfasst das Charakterisieren ein Kategorisieren von Stufenbreiten in eine vorbestimmte Anzahl von Breitenkategorien und/oder ein Kategorisieren gegebenenfalls in der Abfolge enthaltener Angaben über auftretende Stufenhöhen. Die Breitenkategorien umfassen dabei Breiten, die in einem Intervall liegen, das durch eine vorgegebene minimale Stufenbreite und eine vorgegebene maximale Stufenbreite vorgegebenen sein kann; vorzugsweise liegen den einzelnen Kategorien jeweils im Wesentlichen gleich große Intervalle in dieser Weise zugrunde. Gegebenenfalls in der Abfolge enthaltene Angaben über auftretende Stufenhöhen können beispielsweise durch die jeweilige Anzahl übereinanderliegender Bändchenschichten kategorisiert sein, die die betreffende Stufe ausbilden.

Auf diese Weise kann ein Datenumfang eines digitalen Repräsentanten des Oberflächenprofils weiter reduziert und insbesondere das Auffinden weiter beschleunigt werden.

Besonders bevorzugt ist eine Ausführungsform, bei der das Auffinden mit Hilfe eines automatisierten, sukzessiven Vergleichens (z.B. entsprechend einer Stufenanordnung) einzelner durch die Abfolge beschriebener Stufenrichtungen und/oder Stufenbreiten und/oder Stufenhöhen mit Knoten eines Suchbaums erfolgt, mittels dessen die Datenbank vorzugsweise strukturiert ist oder werden kann. Insbesondere vorteilhaft ist es, wenn der Suchbaum ein neuronales Netzwerk umfasst.

Damit kann das Auffinden durch systematische Vergleiche besonders geringer Anzahl erreicht werden, was das Verfahren insbesondere dann beschleunigt, wenn die Datenbank eine große Anzahl an Einträgen umfasst.

Wie erwähnt kann die Trefferwahrscheinlichkeit in der Zuordnung auf Grundlage von bekannten Daten (z.B. Erfahrungswerten), maschinellen Lernschritten und/oder mindestens einem statistischen Verfahren beruhen. Besonders bevorzugt ist eine Ausführungsform, bei der die Trefferwahrscheinlichkeit eine Wahrscheinlichkeit dafür einschließt (bzw. umfasst), dass das Oberflächenprofil tatsächlich erfasst wird, wenn ein Fehler des Fehlertyps auftritt; bei dieser Ausführungsform ist also die zuletzt genannte Wahrscheinlichkeit (die in dieser Schrift als "Oberflächenprofilwahrscheinlichkeit" bezeichnet wird) bei der Zuweisung eines Wertes für die Trefferwahrscheinlichkeit berücksichtigt. Alternativ oder zusätzlich kann die Trefferwahrscheinlichkeit analog vorzugsweise eine Wahrscheinlichkeit dafür einschließen (umfassen), dass das erfasste Oberflächenprofil mindestens einen Messfehler enthält (dass also sein Erfassen auf einem Messfehler beruht) und/oder dafür, dass ein Fehler des (dem Oberflächenprofil zugeordneten) Fehlertyps auf dem Faserverbundwerkstück tatsächlich auftritt; die letzten beiden genannten Wahrscheinlichkeiten werden im Sinne einer besseren Unterscheidbarkeit in dieser Schrift als "Messfehlerwahrscheinlichkeit" bzw. "Auftrittswahrscheinlichkeit" bezeichnet.

Diese Ausführungsformen ermöglichen besonders belastbare, wirklichkeitsnahe Trefferwahrscheinlichkeiten in den Zuordnungen und damit ein besonders zuverlässiges probabilistisches Erkennen von Fehlern einschließlich ihrer Typisierung.

Der durch die Zuordnung gelieferte Fehlertyp und/oder die zugehörige Trefferwahrscheinlichkeit werden vorzugsweise automatisch bestimmt; die jeweiligen Werte können beispielsweise ausgegeben, gespeichert und/oder für mindestens eine automatische Berechnung verwendet werden.

Gemäß einer vorteilhaften Variante umfasst ein erstes erfindungsgemäßes Verfahren ein Ausgeben mindestens eines dem Oberflächenprofileintrag zugeordneten Fehlertyps sowie der zugehörigen Trefferwahrscheinlichkeit auf einem Ausgabegerät. Vorzugsweise wird dabei in Zusammenhang mit dem Fehlertyp und der Trefferwahrscheinlichkeit zudem ausgegeben, an welcher Stelle bzw. Position des Werkstücks das Oberflächenprofil erfasst wurde.

Damit kann ein Anwender den Kontrollfortschritt verfolgen und insbesondere erkennen, wenn ein Fehler an den verlegten Materialbändchen erfasst und ein zugehöriges Pendant in der Datenbank aufgefunden wurde. Darüber hinaus erfährt der Anwender die zugeordnete Trefferwahrscheinlichkeit, und ggf. erhält der Anwender zudem einen Hinweis auf die Position des mutmaßlichen Fehlers. Diese Informationen erleichtern es dem Anwender, Schlüsse für geeignet zu treffende Maßnahmen ziehen.

Besonders bevorzugt ist eine Ausführungsform, bei der das genannte Ausgeben ein Feststellen umfasst, dass die Trefferwahrscheinlichkeit einen vorgegebenen Mindestwert erreicht oder überschreitet; vorzugsweise ist der Mindestwert durch einen Anwender einstellbar.

Dies erlaubt ein Ausgeben von lediglich solchen dem Oberflächenprofil zugeordneten Fehlertypen, die für die Praxis bzw. (bei einer Einstellbarkeit des Mindestwerts) für den jeweiligen Fall relevant sind. Insbesondere kann ein Ausgeben unterdrückt werden, wenn die von dem aufgefundenen Oberflächenprofileintrag gelieferte, dem Oberflächenprofil zugeordnete Trefferwahrscheinlichkeit (für den zugeordneten Fehlertyp) den Mindestwert unterschreitet.

Die Datenbank kann außer dem aufgefundenen noch einen oder mehrere weitere Einträge mit einer bzw. mehreren Zuordnung(en) des Oberflächenprofils zu demselben oder anderen Fehlertyp(en) und zugehörigen Fehlertypwahrscheinlichkeit(en) umfassen:
Insbesondere können der aufgefundene Oberflächenprofileintrag ein erster Oberflächenprofileintrag, der zugeordnete Fehlertyp ein erster Fehlertyp und die zugeordnete Trefferwahrscheinlichkeit eine erste Trefferwahrscheinlichkeit sein. Das Verfahren kann weiterhin ein Auffinden mindestens eines weiteren Oberflächenprofileintrags in der Datenbank umfassen, der eine (weitere) Zuordnung liefert (der also Informationen enthält, die die Zuordnung ermöglichen), nämlich eine Zuordnung des Oberflächenprofils zu mindestens einem weiteren, vom ersten verschiedenen Fehlertyp und mindestens einer weiteren Trefferwahrscheinlichkeit (als einer Wahrscheinlichkeit dafür, dass dem erfassten Oberflächenprofil ein Fehler des mindestens einen weiteren Fehlertyps zugrunde liegt).

Somit wird berücksichtigt, dass verschiedene Fehlertypen zu demselben Oberflächenprofil führen können: Zum einen können die Fehler verschiedenen Fehlertyps in Abschnitten (z.B. entlang gewisser Linien) übereinstimmende Oberflächenprofile aufweisen, zum anderen können Messfehler trotz unterschiedlicher zugrundeliegender Fehlertypen zu übereinstimmenden Oberflächenprofilen führen. Die genannte Variante liefert somit umfassende Information über verschiedene mögliche Fehlertypen, die dem erfassten Oberflächenprofil theoretisch zugrunde liegen könnten, sowie die zugehörigen Trefferwahrscheinlichkeiten. Vorzugsweise werden die mindestens zwei so zugeordneten Fehlertypen mit zugehörigen Trefferwahrscheinlichkeiten wie oben geschildert an einem Ausgabegerät ausgegeben (z.B. wenn die Trefferwahrscheinlichkeiten einen Mindestwert erreichen oder überschreiten).

So erhält ein Anwender einen fundierten Überblick über möglicherweise auf dem Werkstück vorhandene Fehler bestimmter Fehlertypen, und er kann damit und anhand der zugehörigen Trefferwahrscheinlichkeiten die Qualität eines Werkstücks einschätzen und ggf. geeignete Maßnahmen treffen.

Das Verfahren kann weiterhin ein Addieren oder Multiplizieren der ersten und der mindestens einen weiteren Trefferwahrscheinlichkeit umfassen; insbesondere können auf diese Weise Trefferwahrscheinlichkeiten, die den in der Datenbank aufgefundenen Oberflächenprofileinträgen jeweils zugeordnet sind, sukzessive mit jedem Auffinden akkumuliert werden. Das Verfahren kann zudem ein Feststellen umfassen, dass die erhaltene Summe eine vorgegebene Schranke erreicht oder überschreitet.

Bei einem derartigen Erreichen oder Überschreiten kann z.B. eine Suche nach weiteren Oberflächenprofileinträgen in der Datenbank abgebrochen werden. Dieser Ausführungsform liegt der Gedanke zugrunde, dass bei Erreichen bzw. Überschreiten der Schranke die verbleibende Trefferwahrscheinlichkeit für mögliche weitere Fehlertypen so klein ist, dass diese Fehlertypen vernachlässigt werden können, und sie bietet den Vorteil eines besonders schnellen Verfahrens. Insbesondere kann damit die Wahrscheinlichkeit für ein Vorliegen von Fehlern bestimmter Typen abgeschätzt werden, wenn diese Fehler in der Summe noch nicht berücksichtigt sind: Sind beispielsweise in einem speziellen Fall insbesondere Fehler eines Typs T₁ von Interesse und beträgt die Summe zu den Fehlertypen T₂ bis Tₙ gehöriger Trefferwahrscheinlichkeiten bereits 92%, so kann geschlossen werden, dass die Wahrscheinlichkeit für ein Vorliegen eines Treffer des Typs T₁ höchstens 8% beträgt.

Das Verfahren kann dann ferner ein Ausgeben einer Fehlermeldung auf einem Ausgabegerät umfassen, wobei die Fehlermeldung vorzugsweise eine Anzeige der summierten Trefferwahrscheinlichkeiten und zugehörigen Fehlertypen enthält. Vorzugsweise enthält die Fehlermeldung eine Angabe darüber, an welcher Position des Werkstücks das Oberflächenprofil erfasst wurde.

Damit kann ein Anwender darauf hingewiesen werden, dass mit einer durch die Schranke gegebenen Mindestwahrscheinlichkeit allgemein ein Fehler an dem Werkstück vorliegt, und ggf. dessen Position.

Vorzugsweise umfasst ein erstes erfindungsgemäßes Verfahren weiterhin ein Erfassen mindestens eines weiteren Oberflächenprofils, vorzugsweise einer Vielzahl weiterer Oberflächenprofile, zu denen ggf. jeweils analog dem oben Beschriebenen mindestens ein entsprechender Oberflächenprofileintrag automatisch aufgefunden werden kann, der analog eine Zuordnung zu einem Fehlertyp und einer Trefferwahrscheinlichkeit für das weitere Oberflächenprofil liefert.

Auf diese Weise können mindestens ein flächiger Bereich der Oberfläche des Werkstücks an verschiedenen Stellen untersucht und gegebenenfalls Fehler bzw. Fehlertypen in dem Bereich probabilistisch erkannt werden.

Gemäß einer besonders bevorzugten Ausführungsform eines ersten erfindungsgemäßen Verfahrens erfolgen das Erfassen des mindestens einen Oberflächenprofils und das Auffinden des Oberflächenprofileintrags während oder nach einer automatischen Schichtlegung eines automatischen Schichtlegeprozesses.

Ein zweites erfindungsgemäßes Verfahren dient einem Anlegen einer Datenbank, die zu einer Fehleranalyse bei auf einem Faserverbundwerkstück verlegten Materialbändchen geeignet ist. Das Verfahren umfasst ein Bestimmen mindestens eines aus einem Fehlertyp resultierenden Oberflächenprofils. Das Bestimmen kann durch einen Anwender erfolgen und/oder ein Abtasten mindestens eines Oberflächenprofils eines Fehlers des Fehlertyps mit Hilfe einer Sensoreinrichtung umfassen.

Für jedes bestimmte Oberflächenprofil umfasst das zweite erfindungsgemäße Verfahren weiterhin ein automatisches Bestimmen einer (Treffer-)Wahrscheinlichkeit dafür, dass bei einem Erfassen des Oberflächenprofils mit einer Sensoreinrichtung ein Fehler des Fehlertyps vorliegt. Das Bestimmen kann auf Grundlage vorhandener Daten erfolgen, die beispielsweise von einem Anwender als Erfahrungswerte eingegeben und/oder mittels maschinellen Lernens gewonnen wurden, und/oder es kann eine Anwendung mindestens eines statistischen Verfahrens umfassen. Vorzugsweise umfassen derartige Daten Wahrscheinlichkeitswerte dafür, dass ein Fehler des Fehlertyps in der Realität auftritt.

Schließlich umfasst das zweite erfindungsgemäße Verfahren für jedes bestimmte Oberflächenprofil ein Speichern mindestens einer ersten Zuordnung des bestimmten Oberflächenprofils zu dem Fehlertyp und zur Trefferwahrscheinlichkeit als Eintrag einer Datenbank (bzw. einer maschinell verwalteten Datenstruktur, die mit dem mindestens einen Eintrag die Datenbank bildet).

Insbesondere wird durch das zweite erfindungsgemäße Verfahren eine für eine Anwendung des ersten erfinderischen Verfahrens geeignete Datenbank angelegt und so eine geeignete Grundlage für die genannten Vorteile geschaffen. Das erste und das zweite erfindungsgemäße Verfahren ergänzen sich somit.

Demgemäß wird gemäß einer Ausführungsform des ersten erfindungsgemäßen Verfahrens eine Datenbank verwendet, die gemäß einem zweiten erfindungsgemäßen Verfahren angelegt ist. Insbesondere kann das erste erfindungsgemäße Verfahren die Schritte des zweiten erfindungsgemäßen Verfahrens umfassen. Es versteht sich jedoch, dass eine gemäß dem zweiten erfindungsgemäßen Verfahren angelegte Datenbank gespeichert vorliegen und für eine Vielzahl an Untersuchungen verschiedenster Werkstücke nach einer Ausführungsform des ersten erfindungsgemäßen Verfahrens verwendet werden kann.

Gemäß einer bevorzugten Ausführungsform eines zweiten erfindungsgemäßen Verfahrens enthält der in der Datenbank gespeicherte Eintrag eine Charakterisierung des Oberflächenprofils in Form einer Abfolge von Stufenrichtungen und Stufenbreiten. Eine derartige Abfolge als Repräsentant des Oberflächenprofils verbraucht zum einen wenig Speicherplatz, zum anderen kann eine sie später - wie oben beschrieben - mit relativ wenigen Vergleichsschritten in der Datenbank wieder aufgefunden werden, beispielsweise bei Anwendung eines ersten erfindungsgemäßen Verfahrens.

Eine vorteilhafte Ausführungsform eines zweiten erfindungsgemäßen Verfahrens umfasst ein Erzeugen mindestens eines modifizierten Oberflächenprofils durch Modifizieren des bestimmten mindestens einen Oberflächenprofils; das Erzeugen kann insbesondere automatisch gemäß einem vorprogrammierten Algorithmus erfolgen. Für jedes modifizierte Oberflächenprofil umfasst das Verfahren weiterhin ein Bestimmen einer Messfehlerwahrscheinlichkeit dafür, dass das modifizierte Oberflächenprofil von einem Sensorsystems aufgrund eines Messfehlers anstelle des bestimmten Oberflächenprofils erfasst wird, ein Bestimmen einer (Treffer-)Wahrscheinlichkeit dafür, dass bei Erfassen des modifizierten Oberflächenprofils ein Fehler des Fehlertyps vorliegt, und ein Speichern einer Zuordnung des modifizierten Oberflächenprofils zu dem Fehlertyp und zu der Trefferwahrscheinlichkeit (für das modifizierte Oberflächenprofil) als Eintrag der Datenbank.

Das Bestimmen der Messfehlerwahrscheinlichkeit kann automatisch auf Grundlage vorliegender Daten, maschinellen Lernens und/oder mindestens eines statistischen Verfahrens erfolgen.

Das Bestimmen der Trefferwahrscheinlichkeit für das modifizierte Oberflächenprofil kann auf Grundlage der Messfehlerwahrscheinlichkeit und der Trefferwahrscheinlichkeit für das zugehörige (nicht modifizierte) Oberflächenprofil erfolgen, bevorzugt unter Berücksichtigung möglicher Übereinstimmungen des modifizierten Oberflächenprofils mit einem anderen Oberflächenprofil, das aus einem Fehler desselben Fehlertyps resultiert, und möglicher Übereinstimmungen mit modifizierten Oberflächenprofilen eines anderen Oberflächenprofils, das aus einem Fehler desselben Fehlertyps resultiert.

Eine mittels einer derartigen Ausführungsform angelegte, derartige Zuordnungen umfassende Datenbank berücksichtigt mögliche Ungenauigkeiten einer Sensoreinrichtung beim Erfassen eines Oberflächenprofils auf einem Werkstück. Diese Ausführungsform ermöglicht daher ein zuverlässiges Erkennen von Fehlern (insbesondere bei Verwendung durch eine Ausführungsform des ersten erfindungsgemäßen Verfahrens) selbst dann, wenn Messfehler der Sensoreinrichtung vorliegen.

Ein erfindungsgemäßes computerlesbares Speichermedium umfasst Instruktionen, die dazu eingerichtet sind, eine Durchführung eines ersten erfindungsgemäßen Verfahrens gemäß einer der in dieser Schrift beschriebenen Ausführungsformen zu bewirken, wenn ein an eine Prüfvorrichtung angeschlossener Computer bei einer Untersuchung eines Faserverbundwerkstücks die Instruktionen ausführt.

Das Speichermedium enthält somit die Programminformationen, die ein automatisiertes Ausführen der verschiedenen Ausführungsformen des ersten erfindungsgemäßen Verfahrens ermöglichen; daraus ergeben sich die bereits genannten Vorteile.

Eine erfindungsgemäße Prüfeinrichtung zur Kontrolle von Faserverbundwerkstücken umfasst mindestens eine Sensoreinrichtung zum Abtasten mindestens einer Oberfläche eines Werkstücks und mindestens eine Rechnereinheit, dazu eingerichtet ist, zur Untersuchung des Faserverbundwerkstücks ein erstes erfindungsgemäßes Verfahren gemäß einer der in dieser Schrift genannten Ausführungsformen auszuführen; das Erfassen des Oberflächenprofils erfolgt dabei auf Grundlage von Abtastwerten, die die Sensoreinrichtung liefert. Vorzugsweise erlaubt die Prüfeinrichtung dabei ein Ändern der Datenbank (z.B. systemgesteuert und/oder durch einen Anwender).

Eine erfindungsgemäße Anlage zur automatisierten Herstellung von Faserverbundbauteilen, umfasst einen Legekopf zum automatisierten Verlegen von Materialbändchen auf einem Werkstück eines Faserverbundbauteils, eine Sensoreinrichtung zum Erfassen eines Oberflächenprofils des Werkstücks und eine Steuerungseinrichtung zum Steuern des Legekopfes und der Sensoreinrichtung. Die Sensoreinrichtung weist eine Kamera und eine getrennt von der Kamera angeordnete Laserlichtquelle auf, wobei die Kamera an dem Legekopf befestigt ist. Die Steuereinrichtung ist dazu eingerichtet, abhängig von einer Position der Kamera auf dem Werkstück auszulösen, dass die Laserlichtquelle eine Lichtlinie unter einem vorgegebenen Winkel auf eine Oberfläche des Werkstücks projiziert sowie dass die Kamera die Lichtlinie aufnimmt. Die Kamera ist dazu eingerichtet, mittels eines Reglers mindestens einen vorgegebenen Bereich eines aufgenommenen Bildes auszuwerten, daraus ein Höhenprofil zu erstellen und das Höhenprofil der Steuerungseinheit zu übermitteln.

Die erfindungsgemäße Anlage ermöglicht mit der Sensoreinrichtung ein Erfassen eines Oberflächenprofils und bietet damit eine beispielhafte vorrichtungstechnische Grundlage für ein Ausführen eines ersten erfindungsgemäßen Verfahrens gemäß einer der hier beschriebenen Ausführungsformen. Insbesondere kann die Anlage eine Rechnereinheit umfassen, die dazu eingerichtet ist, zur Untersuchung des Werkstücks ein erstes erfindungsgemäßes Verfahren gemäß einer der in dieser Schrift beschriebenen Ausführungsformen auszuführen, wobei das Erfassen des Oberflächenprofils auf einem Abtasten mindestens eines Oberflächenabschnitts durch die Sensoreinrichtung beruht.

Im Folgenden werden Merkmale bevorzugter Ausführungsbeispiele der Erfindung anhand von Zeichnungen näher erläutert. Es versteht sich, dass die schematisch dargestellten einzelnen Elemente und Komponenten auch anders kombiniert und/oder ausgebildet sein können als gezeigt und dass die vorliegende Erfindung gezeigte Merkmale beschränkt ist.

Es zeigen schematisch:
- Figur 1:: eine Abfolge von Schritten gemäß einer Ausführungsform des ersten erfindungsgemäßen Verfahrens; und
- Figur 2:: einen exemplarischen geeigneten Suchbaum zum Auffinden eines Oberflächenstruktureintrags in einer Datenbank.
- Figur 3:: eine Abfolge von Schritten gemäß einem zweiten erfindungsgemäßen Verfahren.

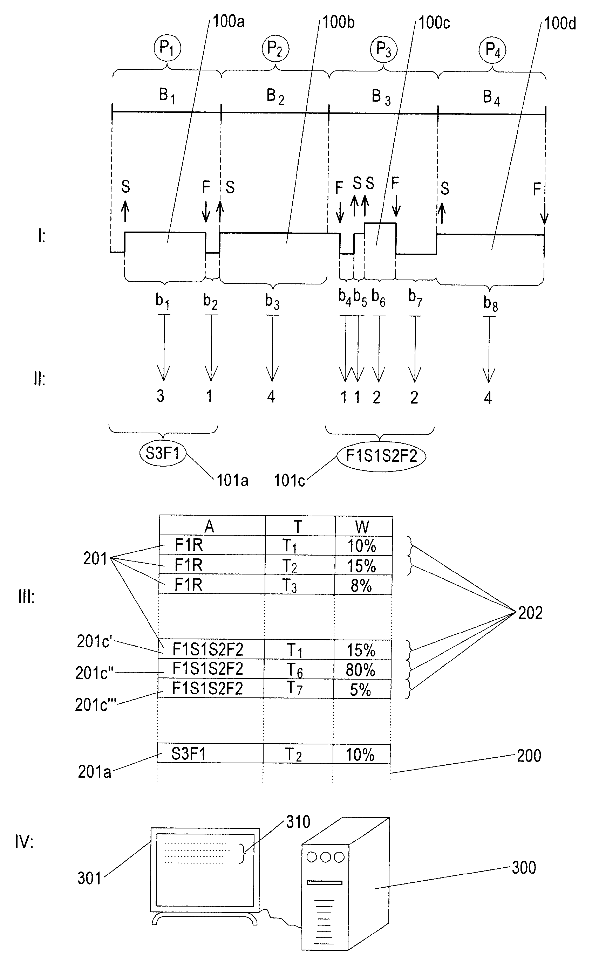

In Figur 1 sind anhand eines fiktiven Beispiels verschiedene Schritte eines ersten erfindungsgemäßen Verfahrens illustriert:
In einem Schritt I werden mehrere Oberflächenstrukturen 100a, 100b, 100c, 100d erfasst, die an verschiedenen nebeneinander verlegten Materialbändchen an den Positionen P₁, P₂, P₃ bzw. P₄ auf einem Werkstück auftreten; eine vorgesehene ideale Verlegung der Bändchen ist schematisch durch die nebeneinander angeordneten Abschnitte B₁, B₂, B₃, B₄ angezeigt, deren Breite jeweils der Breite der Materialbändchen entspricht.

Die erfassten Oberflächenstrukturen werden in einem Schritt II als Abfolge von Stufenrichtungen und Stufenbreiten charakterisiert; die jeweiligen Stufenrichtungen sind dabei in der Figur durch entsprechende Pfeile und die Angaben "S" (für (an)steigende Stufe) bzw. "F" (für (ab)fallende Stufe) gekennzeichnet.

Wie im gezeigten Beispiel zu erkennen ist, weist beispielsweise das Oberflächenprofil 100a je eine ansteigende Stufe und eine abfallende Stufe auf; dazwischen liegt eine Stufenbreite b₁, und auf die abfallende Stufe folgt eine Stufenbreite b₂. Die Oberflächenprofile 100b und 100d hingegen enthalten über die gesamte Bändchenbreite b₃ bzw. b₈ keine Stufen.
Das Oberflächenprofil 100c schließlich weist, wie in der Figur gekennzeichnet, mehrere abfallende und ansteigende Stufen mit jeweiligen Breiten b₄, b₅, b₆ und b₇ auf.

Gemäß der exemplarischen in der Figur 1 illustrierten Charakterisierung werden die festgestellten Stufenbreiten b₁, b₂,... b₈ werden in eine vorgegebene Anzahl von Breitenkategorien eingeteilt. Die Breitenkategorien sind dabei durch geeignete Intervalle vorgegeben. So könnte beispielsweise eine Stufenbreite, die in einem (halboffenen) Intervall von 0mm bis 1,5mm liegt, einer Kategorie 1 zugeordnet werden, ferner eine Stufenbreite in einem (halboffenen) Intervall von 1,5mm bis 3mm einer Kategorie 2 zugeordnet werden usw. Beispielsweise bei einer Bändchenbreite von 6mm ergäben sich hiernach vier mögliche Breitenkategorien; es versteht sich, dass die Einteilung hier lediglich zur Erläuterung beispielhaft angegeben ist und auch anders gewählt sein kann; insbesondere können die Intervalle auch unterschiedliche Längen aufweisen.

Im gezeigten Beispiel wird die Breite b₁ der Kategorie 3 zugeordnet, die Breite b₂ der Kategorie 1, die Breite b₃ der Kategorie 4 usw., wie in der Figur 1 angegeben. Die Breiten b₆ und b₇ werden beide derselben Kategorie 2 zugeordnet, weil sie - wie in der Figur zu erkennen ist - in dem gezeigten Beispiel zwar unterschiedlich sind, aber in einem gemeinsamen der Kategorisierung zugrundeliegenden Intervall liegen.

Den stufenfreien Oberflächenstrukturen 100b und 100d liegt offensichtlich kein Verlegungsfehler zugrunde, weswegen diese Oberflächenstrukturen im dargestellten Beispiel vorzugsweise nicht weiter berücksichtigt werden.

Die Oberflächenstrukturen 100a und 100c werden gemäß den festgestellten, in der Figur 1 angegebenen Stufenrichtungen und -breiten in ihren entsprechenden Stufenanordnungen (im vorliegenden Beispiel wird eine Reihenfolge von links nach rechts zugrundegelegt) durch die Abfolgen 101 a bzw. 101 c charakterisiert; insbesondere ergeben sich die Abfolge "S3F1" für das Oberflächenprofil 100a und die Abfolge "F1S1S2F2" für das Oberflächenprofil 100c.

Vorzugsweise werden die Positionen P₁ und P₃, an denen die Oberflächenprofile 100a bzw. 100c erfasst wurden, mit den Oberflächenprofilen 100a bzw. 100c (bzw. den zugehörigen Abfolgen) verknüpft, beispielsweise in einem Speicher einer Rechnereinheit 300, die dazu eingerichtet sein kann, das Verfahren auszuführen. So können (nach dem Auffinden entsprechender Oberflächenprofileinträge) in einer Anzeige die möglichen Fehlertypen und Trefferwahrscheinlichkeiten den entsprechenden Positionen zugeordnet werden.

In einem Schritt III werden nun zu den durch die Abfolgen 101 a, 101 c charakterisierten Oberflächenprofilen 100a, 100c jeweils zugehörige Oberflächenprofileinträge 201 in einer Datenbank 200 gesucht. Im gezeigten Fall ist diese Datenbank als Tabelle strukturiert, die eine Spalte A mit verschiedenen, jeweils einen Oberflächenprofileintrag charakterisierenden Abfolgen, eine Spalte T mit möglichen Fehlertypen (z.B. "Verdrehung" oder "Überlappung" o.ä.) und eine Spalte W mit zugehörigen Trefferwahrscheinlichkeiten umfasst. Die in der Figur gezeigten Werte für Abfolgen, Anzahlen möglicher Fehlertypen und Trefferwahrscheinlichkeiten sind dabei rein fiktiv und dienen lediglich der Erläuterung des Verfahrens.

Die verschiedenen Oberflächenprofileinträge 201 in dieser Datenbank 200 liefern im gezeigten Beispiel über die Zeilen 202, in denen sie in der Tabelle angeordnet sind, jeweils eine Zuordnung einer möglichen Abfolge (und damit eines möglichen Oberflächenprofils) zu einem Fehlertyp und einer Trefferwahrscheinlichkeit.

Beispielsweise für die im Schritt I erfassten Oberflächenprofile 100a, 100c bzw. die zugehörigen Abfolgen 101 a, 101 c umfasst die Datenbank 200 die Oberflächenprofileinträge 201a, 201c', 201c", 201c'"; insbesondere ist in der Tabelle 200 berücksichtigt, dass dem durch die Abfolge 100c charakterisierten Oberflächenprofil 100c verschiedene Fehlertypen zugrunde liegen können. Die Zeilen, in denen die Oberflächenprofileinträge in der Tabelle angeordnet sind, liefern beispielsweise die möglichen Fehlertypen T₁, T₆ und T₇ für das Oberflächenprofil 100c sowie die zugehörigen Trefferwahrscheinlichkeiten dafür, dass dem Oberflächenprofil 100c, wenn es erfasst wird, tatsächlich ein Fehler des jeweiligen Typs zugrunde liegt; Analoges gilt für das Oberflächenprofil 100a.

Gemäß dem ersten erfindungsgemäßen Verfahren wird im vorgegebenen Beispiel mindestens einer der (vorliegend) drei Oberflächenprofileinträge 201 c', 201 c" und 201 c'" aufgefunden. Bevorzugt ist eine Variante, derzufolge mehrere oder sogar alle jeweils passenden Oberflächenprofileinträge aufgefunden werden.

Dabei können sukzessive die zugehörigen Trefferwahrscheinlichkeiten addiert werden. Bei Erreichen einer (Zwischen)Summe, die eine vorgegebene Schranke erreicht oder überschreitet, kann die Suche nach weiteren (- in diesem Beispiel - zu dem Oberflächenprofil 100c) passenden Oberflächenprofileinträgen abgebrochen und so eine Verfahrensdauer verkürzt werden.

Im gezeigten Beispiel könnte beispielsweise die Suche nach einem Auffinden der Oberflächenprofileinträge 201c' und 201 c" beendet werden, weil ihre summierten Trefferwahrscheinlichkeiten in Höhe von 95% eine Schranke von z.B. 90% überschreiten und verbleibende andere Möglichkeiten für Fehlertypen, die dem Oberflächenprofil 100c zugrunde liegen könnten, zusammen lediglich eine Wahrscheinlichkeit von 5% haben und damit vernachlässigt werden können.

Im Schritt IV werden anhand der Zuordnungen in den aufgefundenen Oberflächenprofileinträgen Angaben über die Oberflächenprofile 101 a, 101 c auf einem Anzeigegerät 301 angezeigt. Eine derartige Anzeige 310 könnte beispielsweise die Information enthalten, dass an der Position P₃ mit einer Trefferwahrscheinlichkeit von 15% ein Fehler des Typs T₁ und mit einer Trefferwahrscheinlichkeit von 80% ein Fehler des Typs T₆ vorliegt, dass an der Position P₃ mit einer Trefferwahrscheinlichkeit von 95% ein Fehler eines der Typen T₁ oder T₆ vorliegt und/oder dass die Wahrscheinlichkeit dafür, dass an der Position P₃ ein Fehler des Typs T₂ vorliegt, maximal 5% beträgt.

Diese Informationen können es einem Anwender erleichtern, geeignete Maßnahmen zu treffen.

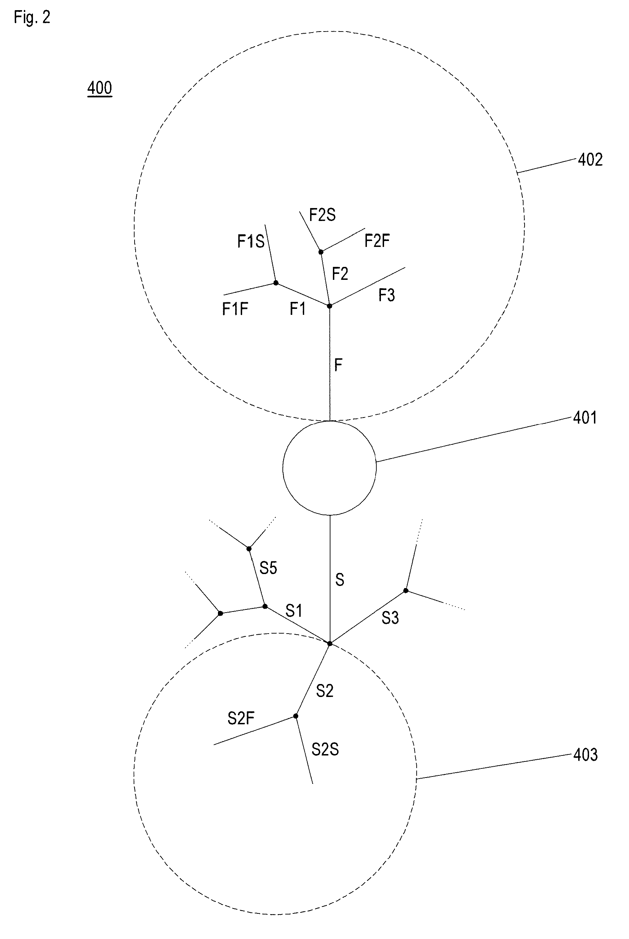

In Figur 2 ist beispielhaft ein geeigneter Aufbau eines Suchbaums 400 dargestellt, mittels dessen die Datenbank vorzugsweise strukturiert ist; die eingetragenen Werte sind auch in dieser Figur rein fiktiv.

Ergibt ein Oberflächenprofil beispielsweise eine Abfolge S2F3, so kann ein Datenbankmanagementsystem beginnend an der Wurzel 401 sukzessive zunächst den Teilbaum 402 aller mit F beginnenden Abfolgen von der weiteren Untersuchung ausschließen, dann die weitere Suche auf den Teilbaum 403 mit Abfolgen beschränken, bei denen auf ein "S" eine 2 folgt usw.. Es versteht sich, dass ein Suchbaum auch anders strukturiert sein kann als angegeben. Vorzugsweise entsprechen die Knoten einem oder mehreren Oberflächenprofileinträgen oder sind mit diesen verknüpft, so dass anhand der Knoten die möglichen Fehlertypen und zugehörigen Trefferwahrscheinlichkeiten erkannt werden können.

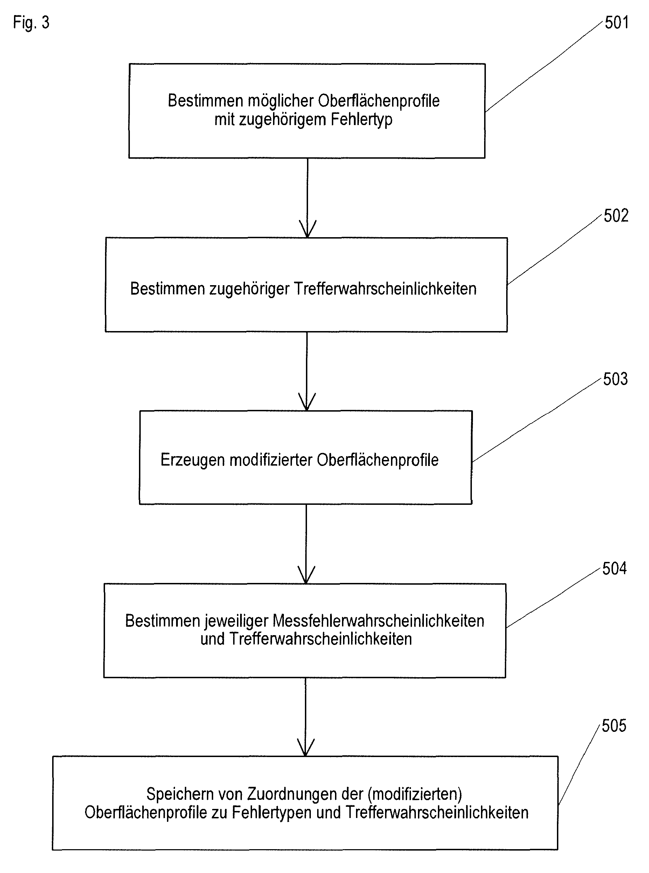

Figur 3 zeigt schematisch eine Abfolge von Verfahrensschritten zum Anlegen einer Datenbank gemäß einer exemplarischen Ausführungsform des zweiten erfindungsgemäßen Verfahrens.

Das Verfahren umfasst einen Schritt 501, in dem mögliche Oberflächenprofile bestimmt werden, die aus einem Fehler eines bestimmten Fehlertyps resultieren können. Wie erwähnt können das Bestimmen und die Zuordnung zu dem jeweiligen Fehlertyp durch einen Anwender erfolgen und/oder ein Abtasten eines Fehlers eines bekannten Fehlertyps durch eine Sensoreinrichtung sowie ein Aufzeichnen erfasster Oberflächenprofile umfassen.

Das Verfahren umfasst weiterhin einen Schritt 502, in dem zu den bestimmten Oberflächenprofilen jeweils Trefferwahrscheinlichkeiten bestimmt werden, also Werte, die den Wahrscheinlichkeiten dafür, dass bei einem Erfassen des Oberflächenprofils mit einer Sensoreinrichtung ein Fehler des Fehlertyps vorliegt, jeweils zugewiesen werden; dieses Bestimmen kann auf Grundlage von bekannten Daten (z.B. aus Erfahrungswerten), mindestens einem statistischen Verfahren und/oder maschinellem Lernen erfolgen.

In einem Schritt 503 werden aus mindestens einem der bestimmten möglichen Oberflächenprofile modifizierte Oberflächenprofile erzeugt.

Für jedes so erzeugte modifizierte Oberflächenprofil werden in einem Schritt 504 eine Messfehlerwahrscheinlichkeit bestimmt (also ein Wert, der der Wahrscheinlichkeiten dafür zugewiesen wird, dass das modifizierte Oberflächenprofil von einer Sensoreinrichtung aufgrund eines Messfehlers anstelle des bestimmten Oberflächenprofils erfasst wird) sowie eine Trefferwahrscheinlichkeiten (ein Wert, der der Wahrscheinlichkeiten dafür zugewiesen wird, dass ein Erfassen des modifizierten Oberflächenprofils jeweils auf den Fehlertyp hinweist).

Schließlich werden in einem Schritt 505 Zuordnungen der bestimmten und der modifizierten Oberflächenprofile jeweils zu Fehlertypen und zugehörigen Trefferwahrscheinlichkeiten in einer Datenstruktur gespeichert, die vorzugsweise von einem geeigneten Datenbankmanagementsystem verwaltet wird und die die Datenbank ergibt.

### Bezugszeichen

- 100a, 100b, 100c, 100d: Oberflächenprofil
- 101a, 101c: Abfolge
- 200: Datenbank
- 201, 201 a, 201c', 201 c", 201c'": Oberflächenprofileintrag
- 202: Zeile
- 300: Rechnereinheit
- 301: Anzeigegerät
- 310: Anzeige
- 400: Suchbaum
- 401: Suchbaumwurzel
- 402,403: Teilbaum
- 501, ..., 505: Verfahrensschritt
- b1, b2, ... b8: Stufenbreite
- B1, B2, B3, B4: Abschnitt
- P1, P2, P3, P4: Position

## Patentansprüche

1. Verfahren zum probabilistischen Erkennen von Fehlern bei auf einem Faserverbundwerkstück verlegten Materialbändchen, wobei das Verfahren umfasst:
Erfassen mindestens eines Oberflächenprofils (100a, 100b, 100c, 100d) eines Werkstücks mit verlegten Materialbändchen; und
Auffinden eines Oberflächenprofileintrags (201, 201a, 201c', 201c", 201c'") in einer Datenbank (200), wobei der Oberflächenprofileintrag eine Zuordnung des erfassten Oberflächenprofils (100a, 100c) zu einem Fehlertyp und zu einer Trefferwahrscheinlichkeit dafür liefert, dass dem erfassten Oberflächenprofil ein Fehler des Fehlertyps zugrunde liegt.

2. Verfahren gemäß Anspruch 1, wobei das Erfassen eines Oberflächenprofils ein Abtasten mindestens eines Oberflächenabschnitts des Werkstücks mit mindestens einer Sensoreinrichtung umfasst.

3. Verfahren gemäß Anspruch 1 oder 2, das ein Charakterisieren des erfassten Oberflächenprofils durch eine Abfolge (101 a, 101c) umfasst, wobei die Abfolge Angaben über bei dem Oberflächenprofil auftretende Stufenrichtungen, Stufenbreiten und/oder Stufenhöhen umfasst, und wobei das Auffinden des Oberflächenprofileintrags auf Grundlage der Abfolge erfolgt.

4. Verfahren gemäß Anspruch 3, wobei das Charakterisieren ein Kategorisieren von Stufenbreiten in eine vorbestimmte Anzahl von Breitenkategorien umfasst.

5. Verfahren gemäß einem der Ansprüche 3 oder 4, wobei das Auffinden mit Hilfe eines sukzessiven Vergleichens einzelner durch die Abfolge (101 a, 101 c) beschriebener Stufenrichtungen und/oder Stufenbreiten und/oder Stufenhöhen mit Knoten eines Suchbaums (400) erfolgt,
wobei der Suchbaum vorzugsweise ein neuronales Netzwerk umfasst.

6. Verfahren gemäß einem der vorhergehenden Ansprüche, wobei die Trefferwahrscheinlichkeit einschließt:
eine Oberflächenprofilwahrscheinlichkeit dafür, dass das Oberflächenprofil bei Auftreten eines Fehlers des Fehlertyps erfasst wird;
eine Messfehlerwahrscheinlichkeit dafür, dass das erfasste Oberflächenprofil auf mindestens einem Messfehler beruht; und/oder
eine Auftrittswahrscheinlichkeit dafür, dass ein Fehler des Fehlertyps auf dem Faserverbundwerkstück auftritt.

7. Verfahren gemäß einem der vorhergehenden Ansprüche, das zudem ein Ausgeben mindestens eines dem Oberflächenprofileintrag zugeordneten Fehlertyps sowie der zugehörigen Trefferwahrscheinlichkeit auf einem Ausgabegerät (310) umfasst, wobei das Ausgeben vorzugsweise ein Feststellen umfasst, dass die Trefferwahrscheinlichkeit einen vorgegebenen Mindestwert erreicht oder überschreitet.

8. Verfahren gemäß einem der vorhergehenden Ansprüche, bei dem der Oberflächenprofileintrag (201c', 201c", 201c'") ein erster Oberflächenprofileintrag, der Fehlertyp ein erster Fehlertyp und die Trefferwahrscheinlichkeit eine erste Trefferwahrscheinlichkeit sind,
und wobei das Verfahren ein Auffinden mindestens eines weiteren Oberflächenprofileintrags (201c', 201c", 201c'") in der Datenbank umfasst, wobei der mindestens eine weitere Oberflächenprofileintrag eine Zuordnung des Oberflächenprofils (100c) zu mindestens einem weiteren, vom ersten verschiedenen Fehlertyp und mindestens einer weiteren Trefferwahrscheinlichkeit dafür liefert, dass dem erfassten Oberflächenprofil ein Fehler des mindestens einen weiteren Fehlertyps zugrunde liegt.

9. Verfahren gemäß Anspruch 8, das ferner umfasst:
ein Summieren der ersten Trefferwahrscheinlichkeit und der mindestens einen weiteren Trefferwahrscheinlichkeit;
ein Feststellen, dass die Summe eine vorgegebene Schranke erreicht oder überschreitet; und
ein Ausgeben einer Ausgabe (310) auf einem Ausgabegerät (301), wobei die Ausgabe (310) vorzugsweise eine Angabe über die summierten Trefferwahrscheinlichkeiten und zugehörigen Fehlertypen umfasst.

10. Computerlesbares Speichermedium mit Instruktionen, die dazu eingerichtet sind, eine Durchführung eines Verfahrens gemäß einem der Ansprüche 1 bis 9 zu bewirken, wenn ein an eine Prüfvorrichtung angeschlossener Computer bei einer Untersuchung eines Faserverbundwerkstücks die Instruktionen ausführt.

11. Prüfeinrichtung zur Kontrolle von Faserverbundwerkstücken, die umfasst:
mindestens eine Sensoreinrichtung zum Abtasten mindestens eines Oberflächenprofils eines Werkstücks; und
mindestens eine Rechnereinheit (300), dazu eingerichtet ist, zur Untersuchung des Faserverbundwerkstücks ein Verfahren gemäß einem der Ansprüche 1 bis 9 auszuführen.

12. Verfahren zum Anlegen einer Datenbank (200) zur Fehleranalyse bei auf einem Faserverbundwerkstück verlegten Materialbändchen, wobei das Verfahren umfasst:
Bestimmen (501) mindestens eines aus einem Fehlertyp resultierenden Oberflächenprofils (100a, 100c);
sowie für jedes bestimmte Oberflächenprofil:
Bestimmen (502) einer Trefferwahrscheinlichkeit dafür, dass bei einem Erfassen des Oberflächenprofils mit einer Sensoreinrichtung ein Fehler des Fehlertyps vorliegt;
Speichern (505) mindestens einer ersten Zuordnung des bestimmten Oberflächenprofils zu dem Fehlertyp und zu der Trefferwahrscheinlichkeit als Eintrag der Datenbank.

13. Verfahren gemäß Anspruch 12, das weiterhin umfasst:
Erzeugen (503) mindestens eines modifizierten Oberflächenprofils durch Modifizieren des bestimmten mindestens einen Oberflächenprofils;
sowie für jedes erzeugte modifizierte Oberflächenprofil:
Bestimmen (504) einer Messfehlerwahrscheinlichkeit dafür, dass das modifizierte Oberflächenprofil von einer Sensoreinrichtung aufgrund eines Messfehlers anstelle des bestimmten Oberflächenprofils erfasst wird;
Bestimmen (504) einer Trefferwahrscheinlichkeit dafür, dass bei Erfassen des modifizierten Oberflächenprofils ein Fehler des Fehlertyps vorliegt; und
Speichern (505) einer Zuordnung des modifizierten Oberflächenprofils zu dem Fehlertyp und zu der weiteren Trefferwahrscheinlichkeit als Eintrag der Datenbank.

14. Anlage zur automatisierten Herstellung von Faserverbundbauteilen, wobei die Anlage umfasst:
einen Legekopf zum automatisierten Verlegen von Materialbändchen auf einem Werkstück eines Faserverbundbauteils;
eine Sensoreinrichtung zum Erfassen eines Oberflächenprofils des Werkstücks; und
eine Steuerungseinrichtung zum Steuern des Legekopfes und der Sensoreinrichtung;
wobei die Sensoreinrichtung eine Kamera und eine getrennt von der Kamera angeordnete Laserlichtquelle umfasst,
wobei die Kamera an dem Legekopf befestigt ist,
wobei die Steuereinrichtung dazu eingerichtet ist, abhängig von einer Position der Kamera auf dem Werkstück ein Projizieren, durch die Laserlichtquelle, einer Lichtlinie unter einem vorgegebenen Winkel auf eine Oberfläche des Werkstücks sowie ein Aufnehmen der Lichtlinie durch die Kamera auszulösen,
und wobei die Kamera eingerichtet ist, mittels eines Reglers mindestens einen vorgegebenen Bereich eines aufgenommenen Bildes auszuwerten, daraus ein Höhenprofil zu erstellen und das Höhenprofil der Steuerungseinheit zu übermitteln.

15. Anlage gemäß Anspruch 14, die mindestens eine Rechnereinheit (300) umfasst, die dazu eingerichtet ist, zur Untersuchung des Werkstücks ein Verfahren gemäß einem der Ansprüche 1 bis 9 auszuführen, wobei das Erfassen des Oberflächenprofils auf einem Abtasten mindestens eines Oberflächenabschnitts durch die Sensoreinrichtung beruht.
